Europäisches Patentamt

European Patent Office

Office européen des brevets

(11) Veröffentlichungsnummer : **0 017 141
B1**

# EUROPÄISCHE PATENTSCHRIFT

(45) Veröffentlichungstag der Patentschrift :
30.09.81

(51) Int. Cl.³ : **C 07 C 39/32, C 07 C 37/72**

(21) Anmeldenummer : 80101571.0

(22) Anmeldetag : 25.03.80

(54) **Verfahren zur Reinigung von Trichlorphenol.**

(30) Priorität : 30.03.79 US 25419

(43) Veröffentlichungstag der Anmeldung :
15.10.80 (Patentblatt 80/21)

(45) Bekanntmachung des Hinweises auf die Patenterteilung : 30.09.81 Patentblatt 81/39

(84) Benannte Vertragsstaaten :
CH DE FR GB NL

(56) Entgegenhaltungen :
DE - A - 2 121 445
GB - A - 1 204 293

CHEMICAL ABSTRACTS Band 84, nr. 19, 1976
Columbus, Ohio, USA
G.A. MARKUS et al. « Formaldehyde purification
of naphtalene with acid catalyst circulation »
Seite 470, Spalte 2, Abstract nr. 135 355z — in
Verbindung mit Chemical Substance Index Band
84, 1976, Seite 2184 CS

(73) Patentinhaber : **L. Givaudan & Cie Société Anonyme
Patentdienst Postfach**
**CH-4002 Basel (CH)**

(72) Erfinder : **Virgilio, Joseph A.**
**14 Evelyn Terrace**
**Wayne, N.J. 07470 (US)**
Erfinder : **Freudewald, Joachim E.**
**18, Waterloo Drive**
**Morris Plains, N.J. 07960 (US)**

(74) Vertreter : **Urech, Peter, Dr. et al**
**Grenzacherstrasse 124 Postfach 601**
**CH-4002 Basel (CH)**

Imprimerie Jouve, 17, rue du Louvre, 75001 Paris, France

## Verfahren zur Reinigung von Trichlorphenol

Die Erfindung betrifft ein neues Verfahren zur Reinigung von 2,4,5-Trichlorphenol, das darauf beruht, dass die hauptsächlichsten Verunreinigungen in diesem Produkt mit Formaldehyd zur Reaktion gebracht werden.

Die konventionelle industrielle Herstellung von 2,4,5-Trichlorphenol beruht auf der Reaktion von 1,2,4,5-Tetrachlorbenzol mit methylalkoholischem oder wässrig methylalkoholischem Natriumhydroxid. Das rohe Produkt, das kommerziell erhältlich ist, besteht zu ungefähr 94 % aus 2,4,5-Trichlorphenol und ungefähr 6 % Verunreinigungen, bei welchen es sich im wesentlichen um Dichlorphenole und Dichlormethoxyphenole handelt.

Das Germicid Hexachlorophen (bis-[3,5,6-Trichlor-2-hydroxyphenyl] methan) wird erhalten durch Kondensation von 2,4,5-Trichlorphenol mit Formaldehyd. Um das Germicid in roher Reinheit zu erhalten, ist es wünschenswert, von einem 2,4,5-Trichlorphenol von hoher Reinheit auszugehen. Da die Dichlorphenole und die Dichlormethoxyphenole, die im kommerziell erhältlichen 2,4,5-Trichlorphenol vorhanden sind, ebenfalls mit Formaldehyd reagieren, ist es wünschenswert, diese vorgängig der oben erwähnten Kondensation zu entfernen.

Es ist nun überraschend und unerwartet, dass gemäss der vorliegenden Erfindung diese hauptsächlichen Verunreinigungen im rohem Produkt (ca. 94 % 2,4,5-Trichlorphenol und ca. 5,5 % Dichlorphenole + Dichlormethoxyphenole) mit Formaldehyd unter Bedingungen zur Reaktion gebracht werden können, bei denen diese unerwünschten 5,5 % Verunreinigungen zu Kondensationsprodukten reagieren, hingegen das 2,4,5-Trichlorphenol nicht zu Hexachlorophen reagiert. Das nicht reagierende 2,4,5-Trichlorphenol kann hierauf von den Kondensationsprodukten abgetrennt werden, worauf 99,5 % iges 2,4,5-Trichlorphenol in hoher Ausbeute anfällt.

Die kritischen Parameter des erfindungsgemässen Prozesses scheinen die Schwefelsäurekonzentration, die Reaktionstemperatur und die Reaktionszeit zu sein.

Das erfindungsgemässe Verfahren bedingt nun die Reaktionsbedingungen sorgfältig zu bestimmen, bei welchen die unerwünschten Verunreinigungen Kondensationsprodukte mit Formaldehyd bilden, das 2,4,5-Trichlorphenol aber nicht.

Die Form, in welcher Formaldehyd verwendet wird, ist nicht kritisch. So sind z.B. eine 37 % wässrige Lösung oder p-Formaldehyd geeignet.

Die Natur oder die Menge Ueberschuss an Formaldehyd scheinen nicht kritisch zu sein. Obschon aus stöchiometrischen Gründen bloss 1 Mol Formaldehyd pro 2 Mol phenolische Verunreinigungen, die kondensiert werden müssen, vorhanden sein muss, wird bevorzugt ein Ueberschuss, beispielsweise ein mehrfacher Ueberschuss an Formaldehyd verwendet, da das Reagens ökonomisch ist und ein Ueberschuss keinen negativen Einfluss auf das Reinigungsverfahren ausübt.

Eine Menge von Formaldehyd, die grösser als 1 Mol pro Mol Verunreinigung beträgt, ist geeignet, eine Menge von 2-5 Mol pro Mol ist bevorzugt. Speziell bevorzugt ist eine Menge von 3 Mol pro Mol Verunreinigung.

Recht kritisch scheint die Konzentration der Schwefelsäure zu sein. Im Falle von 50 %iger oder noch verdünnterer Schwefelsäure ergeben sich geringere Ausbeuten an 2,4,5-Trichlorphenol und das Reinigungsverfahren ist nicht besonders ergiebig.

Wenn hingegen die Schwefelsäurekonzentration 80 % oder noch grösser ist, reagiert das 2,4,5-Trichlorphenol rasch mit dem Formaldehyd, d.h., die Ausbeute an 2,4,5-Trichlorphenol ist niedrig, und das Reinigungsprozedere zeichnet sich ebenfalls nicht durch hohe Wirksamkeit aus.

Ganz im Gegensatz dazu stehen die Resultate, die mit Schwefelsäurekonzentrationen zwischen 55 und 75 % erhalten werden. Hier ergibt sich eine überraschende Selektivität, indem das Formaldehyd primär mit dem Dichlorphenol und den Methoxydichlorphenolen reagiert, hingegen nicht mit dem 2,4,5-Trichlorphenol. Vorzugsweise wird in der Mitte des obigen Bereiches gearbeitet, nämlich bei Konzentrationen zwischen 60 und 70 %.

Der Temperaturbereich ist von geringerem Einfluss als dies im Falle der Säurekonzentration festgestellt wird. Allerdings sollte auch dieser sorgfältig kontrolliert werden, um die Ausbeute an reinstmöglichem Produkt zu maximalisieren. Bei Temperaturen unterhalb 70 °C wird eine langsamere Reaktion zwischen den zu entfernenden Verunreinigungen und dem Formaldehyd festgestellt, und das erhaltene 2,4,5-Trichlorphenol ist etwas weniger rein.

Bei Temperaturen oberhalb 90 °C scheint die Reaktion weniger selektiv zu sein, und es werden ebenfalls geringere Ausbeuten an 2,4,5-Trichlorphenol beobachtet. Der bevorzugte Temperaturbereich ist deshalb derjenige zwischen 70 und 90 °C. Speziell bevorzugt ist das Arbeiten in der Mitte dieses Bereiches, nämlich zwischen 75 und 85 °C.

Die Reaktionsdauer sollte so lang gewählt werden, bis alle Verunreinigungen mit Formaldehyd reagiert haben. In einem bevorzugten Aspekt beträgt diese Zeit 5-8 Stunden. Aus ökonomischen Gründen ist es jedoch angezeigt, den Reaktionsablauf analytisch zu verfolgen, beispielsweise mittels Gaschromatographie.

Das gereinigte 2,4,5-Trichlorphenol kann von den schwereren Kondensationsprodukten durch bekannte Methoden, beispielsweise durch Extraktion und/oder Destillation abgetrennt werden.

Es existieren eine ganze Reihe geeigneter Extraktionsmittel, die Trichlorphenol auflösen, die hingegen für die weniger löslichen Bis-phenole keine geeigneten Lösungsmittel darstellen. Geeignet sind zu diesem Zweck z.B. Alkane, wie Pentan, Hexan, Heptan, etc.

Bevorzugt ist, das niedriger siedende Trichlorphenol von den höher siedenden Kondensationsprodukten durch Destillation abzutrennen, vorzugsweise durch Wasserdampfdestillation (bei Atmosphärendruck oder bei vermindertem Druck [d.h. Vakuumdampfdestillation]).

Weiter unten folgen eine Reihe Beispiele, die die bevorzugten Aspekte der vorliegenden Erfindung illustrieren. Diese Beispiele sollen illustrierend, nicht aber limitierend verstanden werden. Sie sollen äquivalente oder naheliegende Ausdehnungen, die für den Fachmann selbstverständlich sind, ebenfalls umfassen.

Die Reinheit des 2,4,5-Trichlorphenols wurde mittels Chromatographie in Dampfphase unter Verwendung eine 1/8 in (1″ = 2,54 cm) × 6 Fuss (1 Fuss = 30,48 cm) rostfreien Stahlkolonne, die mit 4 % FFAP® (1) bepackt war, auf 100 × 120 mesh (2) Chromsorb W® (3), säuregewaschen, mit DMCS (4) bestimmt. Ein Flammenjonisationsdetektor wurde benützt.

Das zu reinigende 2,4,5-Trichlorphenol stellte übliches kommerziell erhältliches technisches Material dar. Dessen Reinheit war die folgende (durch Gaschromatographie bestimmt) :

| 2,4,5-Trichlorphenol | 94,0 ± 0,2 % |
| 2,4/2,5-Dichlorphenol | 1,0 ± 0,8 % |
| 2,3,6/2,4,6-Trichlorphenol | 0,3 ± 0,3 % |
| 3,4-Dichlorphenol | 0,1 ± 0,1 % |
| 4,5-Dichlor-2-methoxyphenol | |
| 2,5-Dichlor-4-methoxyphenol | 4,6 ± 0,7 % |
| 2,4-Dichlor-5-methoxyphenol | |

Wenn im folgenden von 2,4,5-Trichlorphenol (TCP), technischer Reinheitsgrad, gesprochen wird, bezieht sich dies auf ein kommerzielles Produkt, das im wesentlichen den obigen oder einen vergleichbaren Reinheitsgrad aufweist, also ungefähr zu 94 % aus 2,4,5-Trichlorphenol besteht.

(1) Free fatty acid packing, d.h. Packung zur Trennung von freien Fettsäuren, ein Carbowax (3)-Substrat, enthaltend substituierte Terphthalsäure.

(2) Siebgrösse.

(3) Trägermaterial für die Gaschromatographie, insbesondere aus Diatomeenerde.

(4) Dimethylchlorsilan, Silylierungsmittel, das benützt wird, um Chromosorb-(Diatomeenerde) Packungen zu deaktivieren.

Beispiel 1

903 g 93 %ige Schwefelsäure wurden verdünnt, indem diese langsam zu 347 g kaltem Wasser [unter Rühren und Kühlen] gegeben wurden. Es wurde TCP, technisch reines Material, zugegeben und das Reaktionsgemisch auf eine Temperatur von 80° erhitzt und bei dieser Temperatur gehalten.

Ueber einen Zeitraum von 4 Stunden wurden 14,0 g einer 37 %igen wässrigen Formaldehydlösung zugegeben. Nach der Zugabe wurde das Reaktionsgemisch 2 weitere Stunden bei 80° gehalten.

Durch Zugabe von ungefähr 600 ml Wasser wurde das Reaktionsgemisch verdünnt und das Produkt mittels Dampfdestillation isoliert.

Es wurden 206,5 g 2,4,5-Trichlorphenol in einer Reinheit von 99,5 % erhalten. Die Ausbeute betrug 87,7 % an 2,4,5-Trichlorphenol des Ausgangsmaterials.

Die Analyse des gereinigten Produkts ergab folgende Werte :

| 2,4,5-Trichlorphenol | 99,5 |
| 2,4/2,5-Dichlorphenol | — |
| 2,3,6/2,4,6-Trichlorphenol | 0,3 |
| 3,4-Dichlorphenol | — |
| 4,5-Dichlor-2-methoxyphenol | |
| 2,5-Dichlor-4-methoxyphenol | 0,2 |
| 2,4-Dichlor-5-methoxyphenol | |

Beispiel 2

Das Beispiel 1 wurde wiederholt, wobei anstelle der 14 g 37 %igen wässrigen Formaldehydlösung 5 g p-Formaldehyd zum Einsatz gelangten. Dieses p-Formaldehyd wurde portionenweise innert 30 Minuten zugegeben.

Es wurden 200,9 g 2,4,5-Trichlorphenol in einer Reinheit von 99,6 % (Ausbeute 85,5 %) erhalten.

Beispiel 3

Beispiel 1 wurde wiederholt, mit dem Unterschied, dass nun 21 g wässriges Formaldehyd verwendet wurden.

Es wurden 200,9 g 99,7 %ig reines 2,4,5-Trichlorphenol (Ausbeute 85,5 %) erhalten.

Beispiel 4

Das Verfahren des Beispiels 2 wurde wiederholt, wobei aber anstelle der Dampfdestillation von einer heissen Heptanextraktion Gebrauch gemacht wurde.

Es wurden 190,6 g 98,3 % reines 2,4,5-Trichlorphenol erhalten (Ausbeute 91,1 %).

Beispiel 5 (Vergleichsbeispiel)

Das Verfahren des Beispiels 2 wurde wiederholt, wobei aber die Reaktionstemperatur 100 °C betrug. Es wurden 167,1 g 99,6 % reines 2,4,5-Trichlorphenol erhalten (Ausbeute 71,0 %). Die Ausbeute beträgt weniger als diejenige des Beispiels 1, was darauf zurückzuführen ist, dass wie vorne gesagt, Temperaturen > 90 °C für das vorliegende Verfahren weniger geeignet sind.

Beispiel 6 (Vergleichsbeispiel)

Das Verfahren des Beispiels 2 wurde wiederholt, mit dem Unterschied, dass nun von einer Schwefelsäurekonzentration von 50 % Gebrauch gemacht wurde. Unter diesen Bedingungen war die Reinheit des erhaltenen Produktes bloss 98,2 %. Diese niedrigere Ausbeute ist auf die geringere Konzentration der Säure zurückzuführen.

Beispiel 7

Das Verfahren des Beispiels 1 wurde wiederholt und der Reaktionsablauf durch Gaschromatographie verfolgt. Auf diese Art konnte eine geringere Reinheit festgestellt werden (bedingt durch die kurzen Reaktionszeiten) :

Reinheit des 2,4,5-Trichlorphenols nach 1 Stunde : 94,7 % ;

Reinheit des 2,4,5-Trichlorphenols nach 2 Stunden : 95,1 % ;

Reinheit des 2,4,5-Trichlorphenols nach 4 Stunden : 98,7 % ;

Reinheit des 2,4,5-Trichlorphenols nach 6 Stunden : 99,5 %.

Beispiel 8

Das Verfahren des Beispiels 1 wurde wiederholt und das 2,4,5-Trichlorphenol mittels Vakuumdampfdestillation abgetrennt.

Die Ausbeute betrug 208,0 g an 99,3 % reinem Produkt (88,5 % Ausbeute).

Beispiel 9 (Vergleichsbeispiel)

Das Verfahren des Beispiels 1 wurde wiederholt, wobei aber von einer Schwefelsäure Gebrauch gemacht wurde, die 80 %ig war. Dabei wurde festgestellt, dass das 2,4,5-Trichlorphenol mit dem Formaldehyd zu einem Bis-phenol reagierte. Das Beispiel dient zur Illustrierung der Tatsache, dass zu hohe Säurekonzentrationen für das Verfahren untauglich sind.

**Ansprüche**

1. Verfahren zur Reinigung von 2,4,5-Trichlorphenol von technischem Reinheitsgrad, dadurch gekennzeichnet, dass mann dieses technisch reine 2,4,5-Trichlorphenol mit Formaldehyd in Anwesenheit von 55-75 %iger Schwefelsäure bei Temperaturen zwischen 70 und 90 °C behandelt und das gereinigte 2,4,5-Trichlorphenol aus dem Reaktionsgemisch abtrennt.

2. Verfahren nach Anspruch 1, dadurch gekennzeichnet, dass man 60-70 %ige Schwefelsäure benützt.

3. Verfahren nach Anspruch 1 oder 2, dadurch gekennzeichnet, dass die Reaktionstemperatur zwischen 75 und 85 °C liegt.

4. Verfahren nach einem der Ansprüche 1-3, dadurch gekennzeichnet, dass das gereinigte 2,4,5-Trichlorphenol mittels Destillation oder Extraktion abgetrennt wird.

5. Verfahren nach Anspruch 4, dadurch gekennzeichnet, dass das gereinigte 2,4,5-Trichlorphenol durch Wasserdampfdestillation isoliert

wird.

6. Verfahren nach einem der Ansprüche 1-5, dadurch gekennzeichnet, dass

a) 2 bis 5 molare Aequivalente von Formaldehyd,

b) 60-70 %ige Schwefelsäure,

c) eine Reaktionstemperatur von 75-85 °C,

d) eine Dampfdestillation zwecks Isolierung des gereinigten 2,4,5-Trichlorphenols

verwendet werden.

7. Verfahren nach Anspruch 6, dadurch gekennzeichnet, dass die Reaktionszeit 5-8 Stunden beträgt.

8. Verfahren nach Anspruch 6 oder 7, dadurch gekennzeichnet, dass man wässriges Formaldehyd benützt.

9. Verfahren nach Anspruch 6 oder 7, dadurch gekennzeichnet, dass man p-Formaldehyd verwendet.

**Claims**

1. Process for the purification of 2,4,5-trichlorophenol of technical degree of purity, characterised in that this technically pure 2,4,5-trichlorophenol is treated with formaldehyde in the presence of 55-75 % sulphuric acid at temperatures between 70 and 90 °C and the purified 2,4,5-trichlorophenol is separated from the reaction mixture.

2. Process according to Claim 1, characterised in that 60-70 % sulphuric acid is used.

3. Process according to Claim 1 or 2, characterised in that the reaction temperature lies between 75 and 85 °C.

4. Process according to one of Claims 1-3, characterised in that the purified 2,4,5-trichlorophenol is separated by means of distillation or extraction.

5. Process according to Claim 4, characterised in that the purified 2,4,5-trichlorophenol is isolated by steam distillation.

6. Process according to one of Claims 1-5, characterised in that there are used :

a) 2 to 5 molar equivalents of formaldehyde,

b) 60-70 % sulphuric acid,

c) a reaction temperature of 75-85 °C,

d) a steam distillation for the purpose of isolating the purified 2,4,5-trichlorophenol.

7. Process according to Claim 6, characterised in that the reaction time amounts to 5-8 hours.

8. Process according to Claim 6 or 7, characterised in that aqueous formaldehyde is used.

9. Process according to Claim 6 or 7, characterised in that p-formaldehyde is used.

**Revendications**

1. Procédé pour purifier le 2,4,5-trichlorophénol à l'état de pureté technique, caractérisé en ce que l'on traite ce 2,4,5-trichlorophénol à pureté technique par le formaldéhyde en présence d'acide sulfurique à une concentration de 55 à

·75 % à des températures de 70 à 90 °C et on sépare le 2,4,5-trichlorophénol purifié du mélange de réaction.

2. Procédé selon la revendication 1, caractérisé en ce que l'on utilise un acide sulfurique à une concentration de 60 à 70 %.

3. Procédé selon la revendication 1 ou 2, caractérisé en ce que la température de réaction se situe entre 75 et 85 °C.

4. Procédé selon l'une des revendications 1 à 3, caractérisé en ce que le 2,4,5-trichlorophénol purifié est séparé par distillation ou extraction.

5. Procédé selon la revendication 4, caractérisé en ce que le 2,4,5-trichlorophénol purifié est isolé par distillation à la vapeur d'eau.

6. Procédé selon l'une des revendications 1 à 5, caractérisé en ce que :

a) on utilise de 2 à 5 équivalents molaires de formaldéhyde

b) on utilise de l'acide sulfurique à une concentration de 60 à 70 %,

c) on observe une température de réaction de 75 à 85 °C,

d) on isole le 2,4,5-trichlorophénol purifié par distillation à la vapeur.

7. Procédé selon la revendication 6, caractérisé en ce que la durée de réaction est de 5 à 8 heures.

8. Procédé selon la revendication 6 ou 7, caractérisé en ce que l'on utilise du formaldéhyde aqueux.

9. Procédé selon la revendication 6 ou 7, caractérisé en ce que l'on utilise du paraformaldéhyde.